# EUROPEAN PATENT APPLICATION

(11) **EP 3 165 153 A1**
(43) Date of publication of application: **10.05.2017**
(21) Application number: 15193169.8
(22) Date of filing: 05.11.2015
(51) Int. Cl.: A61B 5/00, A61B 34/20, A61B 90/00, A61K 49/00, G06T 7/00

(54) **SYSTEM FOR FLUORESCENCE AIDED SURGERY**

(71) Applicant: Deutsches Krebsforschungszentrum Stiftung des Öffentlichen Rechts, 69120 Heidelberg (DE); Ruprecht-Karls-Universität Heidelberg, 69117 Heidelberg (DE)
(72) Inventor: Maier-Hein, Lena, 69120 Heidelberg (DE); Wild, Esther, 69115 Heidelberg (DE)
(74) Representative: Lucke, Andreas

(57) **Abstract**

Disclosed herein is a system for fluorescence aided surgery. The system comprises a storage medium or a data connection with a storage medium storing a 3D data set (28), in particular a medical image, of a patient (10), wherein information regarding a spatial position of one or more fluorescence markers (12) within the body of said patient (10) is included in or associated with said 3D patient data set, a visualization tool (66, 58; 72) allowing for augmenting a view or an image of said patient's body (10) with information derived from said 3D patient data set (28), and an apparatus (64, 74) for detecting fluorescence from said one or more fluorescence markers (12) provided in said patient's body (10). The system further comprises a computation device adapted to derive a spatial relationship between said visualization tool (66, 72) and the patient's body (10), based at least in part on said detected fluorescence of said one or more markers (12), said information regarding said spatial position of said one or more fluorescence markers (12) within the body (10) of said patient included in or associated with said 3D patient data set (28), and a known spatial relationship between the apparatus (64, 74) for detecting fluorescence and said visualization tool (66, 72).

## Description

### FIELD OF THE INVENTION

The present invention is in the field of surgical systems. In particular, the present invention relates to a system for fluorescence aided surgery. The invention also relates to a method of augmenting a view of an image of a patient's body.

### BACKGROUND OF THE INVENTION

A dual-mode stereo imaging system for tracking and controlling in surgical and interventional procedures is known from WO 2013/158636 A1. This prior art system includes a device that deploys fluorescent material on an organ under surgery and on a surgical tool. The system further comprises a light source for emitting light in the visual spectrum, a fluorescence light source for emitting at the excitation wavelength of the fluorescent material, and an image acquisition and control element that controls the visual light source and the fluorescent light source and captures and digitizes the resulting visual images and fluorescent images. The system further includes an image-based tracking module that applies image processing to the visual and fluorescent images such as to detect fluorescence markers on the organ and on the surgical tool. Using stereo image formation and triangulation, the three-dimensional coordinates of the fluorescence markers can be extracted. These 3D-coordinates are then used by a robot motion control algorithm in an open-loop or close-loop architecture. Any error between the tool position and the marker position is calculated and used to generate a desired tool displacement. By combining visual images and fluorescent images, the image-based tracking algorithms can be made more robust.

WO 2014/145606 discloses fluorescent silica-based nanoparticles for precise detection, characterization, monitoring and treatment of a disease such as cancer. In order to target a specific cell type, each nanoparticle is conjugated with a ligand, which is capable of binding to a cellular component, e.g. the cell membrane or intracellular components associated with the specific cell type, such as a tumor marker or a signaling pathway intermediate. To permit the nano particle to be detectable not only by fluorescence imaging, but also other imaging techniques, the nano particle may also be conjugated with a contrast agent, such as a radionuclide. Instrument tracking in computer-assisted surgery using needle-based markers is generally known and e.g. described in Maier-Hein, L; Tekbas, A; Seitel, A; Pianka, F; Müller, SA; Satzl, S; Schawo, S; Radeleff, B; Tetzlaff, R; Franz, AM; Müller-Stich, BP; Wolf, I; Kauczor, HU; Schmied, BM; Meinzer, HP. (2008); "In-vivo accuracy assessment of a needle-based navigation system for CT-guided radiofrequency ablation of the liver"; Med Phys, 35(12), 5385-5396*.* Further, in computer-assisted surgery, it is known to visualize anatomical data by augmented reality, as is for example described in US 13/891,310 and in Müller, M et al. (2013); "Mobile augmented reality for computer-assisted percutaneous nephrolithotomy"; Int J Cars 8(4), 663-675*.*

### SUMMARY OF THE INVENTION

The problem underlying the invention is to provide a computer assisted surgery system which combines a low invasiveness with a robust and reliable behavior. This problem is solved by a system according to claim 1. The problem is also solved by a method according to claim 13. Preferable embodiments are described in the dependent claims.

The system for fluorescence aided surgery according to the invention comprises a storage medium or a data connection with a storage medium storing a 3D data set, in particular a medical image of a patient, wherein information regarding a spatial position of one or more fluorescence markers within the body of said patient is included in or associated with said 3D patient data set. The system further comprises a visualization tool allowing for augmenting a view or an image of said patient's body with information derived from said 3D patient data set, and an apparatus for detecting fluorescence from said one or more fluorescence markers provided in said patient's body. Finally, the system comprises a computation device adapted to derive a spatial relationship between said visualization tool and the patient's body, based at least in part on
- said detected fluorescence of said one or more markers,
- said information regarding said spatial position of said one or more fluorescence markers within the body of said patient included in or associated with said 3D patient data set, and
- a known spatial relationship between the apparatus for detecting fluorescence and said visualization tool.

A key ingredient of the invention is hence a stored 3D patient data set which includes information regarding a spatial position of one or more fluorescence markers within the body of the patient. The 3D data set is typically based on a medical image, such as a computed tomography (CT) image, a magnetic resonance (MR) image or an ultrasound (US) image, which may be taken prior to the surgery, and which could be used for planning the surgery. However, the medical image could also be taken during the surgery. The system further comprises an apparatus for detecting fluorescence from said one or more fluorescence markers provided in the patient's body, such that the fluorescence markers can be detected under surgery. Based on a known spatial relationship between the apparatus for detecting fluorescence and the visualization tool, and based on the information regarding the spatial position of said one or more fluorescence markers within the body of said patient as included in or associated with the 3D patient data set, a spatial relationship between the visualization tool and the patient's body can be calculated. This then allows for combining the view or image of the actual patient's body with information from the 3D patient data set, and thereby to augment the view or image accordingly in a way which is referred to as "augmented reality" in the art, for example by displaying organs at risk or structures that are covered by tissue, blood or surgical instruments or are too small or too similar to the surrounding tissue and hence cannot be seen or discerned with the visualization tool alone.

Importantly, the system of the invention generally allows for augmenting a view or an image of the patient's body with information derived from said 3D patient data set without having to employ traditional fiducials, such as needles. This allows for a lesser degree of invasiveness, since no needles or the like have to be introduced into the body. In fact, in some embodiments described below, the fluorescence markers may even be simply injected and, if appropriately functionalized, will automatically be accumulated in certain areas of the patient's body. But even if the fluorescence markers are implanted in a surgical step, the intervention is typically less invasive than the implantation of conventional needles or the like. Moreover, fluorescence markers tend to interfere less with the surgical intervention than conventional needle-based markers. However, in some embodiments needle-based markers may nevertheless be used in combination with the fluorescence markers, which still provides an improvement over traditional systems.

Preferably, the fluorescence marker emits light in the near infrared (NIR) spectrum. In the present disclosure, the NIR spectral range shall cover the wavelengths from 780 nm to 3 µm. Since the absorption of NIR light in tissue is less pronounced than that of visual light, NIR fluorescence markers can even be detected if they are covered by tissue, smoke (e.g. resulting from surgical coagulation) or blood. This makes the operation of the system particularly robust and reliable.

In a preferred embodiment, the fluorescence marker is biodegradable, thereby eliminating the need to remove the marker after surgery. This is a further advantage over traditional needle-based markers, which may need to be removed after surgery, thereby adding to the invasiveness and complexity of the surgical intervention.

In one embodiment, the visualization tool may comprise a camera and a display for displaying images taken with said camera, augmented with information derived from the 3D data set. However; in alternative embodiments, the visualization tool may be formed by glasses that allow a direct view onto said patient's body under surgery, but at the same time allow for displaying information derived from said 3D patient data set as what is known in the field as "augmented reality". In a yet further embodiment, the visualization tool may be a projector for projecting images or visual information onto a patient's body.

Preferably, the system further comprises at least one fluorescence marker for placing in the body of the patient. Herein, the fluorescence marker may further include a contrast agent or radioactive tracer for medical imaging, in particular for CT, positron emission tomography (PET), single-photon emission computed tomography (SPECT), MR imaging, ultrasound or photoacoustic imaging. Using such contrast agent or radioactive tracer, the information regarding the spatial position of the fluorescence markers within the body of said patient can be readily obtained, typically upon generating the 3D data set in a medical imaging procedure such as CT, PET, SPECT, MR ultrasound or photoacoustic imaging.

Preferably, the fluorescence marker further comprises a ligand or functional group which is selected from a group consisting of peptide, protein, biopolymer, synthetic polymer, anti-gene, anti-body, microorganism, virus, receptor, enzyme, hormone, chemical compound, toxin, surface modifier and combinations thereof. This way, the fluorescence marker can be functionalized and can be used to characterize and monitor diseased cells such as cancer cells in a way described e.g. in WO 2014/145606 A1. In a particularly preferable embodiment, the ligand is a ligand that targets a prostate-specific membrane anti-gene (PSMA), which is a membrane-bound receptor which is highly upregulated in all stages of most types of prostate cancer.

When the fluorescence marker is functionalized by an appropriate ligand or functional group, it may upon application, such as injection, automatically accumulate in diseased tissue, such as cancer tissue, which is then to be removed during surgery. However, the invention is not limited to applications where the fluorescence markers are directly associated with tissue to be excised during surgery. And even given the case, the purpose of the fluorescence markers is not simply to visualize tissue to be excised, but it is also used for correlating the view or image of the patient's body with information of said 3D patient data set and for augmenting the former with the latter.

In a preferred embodiment, the fluorescence marker is included in a liquid for injection to the patient's body. For example, the liquid including the fluorescence marker may be injected subdermally, peritumorally, intravenously, subcutaneously, intramuscularly or transdermally. However, in other embodiments the marker may be simply administered orally. For this purpose the marker could be included in a pill to be swallowed by the patient.

In an alternative embodiment, the fluorescence marker is provided with an adhesive agent or binder, in particular a biodegradable adhesive agent or binder, and preferably a fibrin adhesive agent or cyanoacrylate. According to this embodiment, the fluorescence marker can be attached to certain body parts using said adhesive agent or binder, referred to simply as "adhesive" in the following, rather than having the fluorescence marker automatically accumulate in certain tissue regions upon administering as in the previous embodiment. In this case too, it is necessary that information regarding the spatial position of the fluorescence markers within the body of the patient is included in or associated with the 3D patient data set. This can readily be achieved if the fluorescence marker includes a contrast agent or radioactive tracer as described above, and if their 3D patient data set corresponds to a medical image that has been taken after attaching said fluorescence markers using the adhesive. However, since in this embodiment, the fluorescence markers can be manually placed at desired positions, it is also possible to "manually" select the spatial positions both in the actual patient's body and in the 3D data set, as will be explained with reference to a specific embodiment below.

In a particularly preferred embodiment, the fluorescence marker comprises nanoparticles comprising a compound of the medical imaging contrast agent or radioactive tracer and a fluorescent dye. Herein, the nanoparticles may have a diameter between 1 and 30 nm, preferably between 1 and 10 nm. Moreover, these nanoparticles may be functionalized by means of ligands as described above.

In a preferred embodiment, the system comprises a double barrel syringe, one barrel containing a fluorescence marker and one barrel containing said adhesive optionally mixed using a three way cock. The fluorescence marker and the adhesive can then be delivered simultaneously from the individual barrels and mixed with each other upon administering.

Preferably, the aforementioned apparatus for detecting fluorescence from said one or more fluorescence markers comprises one or more of a laparoscopic fluorescence imaging device, a fluorescence imaging camera or fluorescence imaging glasses.

Preferably, the computation device is adapted to derive said spatial relationship between the visualization device and the patient's body based on a 2D/3D registration algorithm, such as "inside-out-tracking" that is based on a 2D to 3D registration, in which a 2D image of one or more fluorescence markers is correlated with known 3D positions of said fluorescence markers in said 3D patient data set. This type of tracking is generally described e.g. in Baumhauer et. al "Soft tissue navigation for laparoscopic partical nephrectomy", Int J CARS 3(3): 307-314 2008*,* included herein by reference, and shall not be repeated herein.

In addition or alternatively, the computation device may be adapted to derive said spatial relationship between the visualization device and the patient's body by means of a 3D/3D registration algorithm, in which 3D information of the location of said fluorescence markers is obtained using said apparatus for detecting fluorescence, and said 3D information is matched with known 3D positions of said fluorescence markers in said 3D patient data set. An example of this type of tracking is likewise described e.g. in Baumhauer et. al "Soft tissue navigation for laparoscopic partical nephrectomy", Int J CARS 3(3): 307-314 2008*,* included herein by reference, and shall not be repeated herein.

In a preferred embodiment, the aforementioned apparatus for detecting fluorescence comprises two cameras placed at a distance for obtaining stereo data. This way, a 3D distribution of fluorescence markers can be obtained under surgery and matched with the 3D distribution of fluorescence markers associated with the 3D patient data set. The 3D point correspondences can then be used to guide a 3D deformable registration.

In a preferred embodiment, the apparatus for detecting fluorescence is adapted to analyze shadows and/or motions and to derive 3D positions of the fluorescence markers and/or the organ surface therefrom in a way generally described e.g. in Maier-Hein et al. "Optical techniques for 3d surface reconstruction in computer-assisted laparoscopic surgery ", Medical image analysis, 17(8): 974-966, 2013*,* or to measure 3D positions of the fluorescence markers based on time of flight measurements, in a way generally described in the same article.

Preferably, the system is further adapted for augmenting the view or image of said patient's body with one or more of
- target structures, such as tumors, which have been previously marked in the 3D patient data set,
- structures at risk, which are to be saved from injuries, in particular blood vessels or nerves,
- landmarks assisting the surgeon in orientation, in particular organs or bones,
- structures which are covered by tissue,
- fluorescence markers, in particular markers with fluorescence outside the visible part of the spectrum and/or markers that are covered with tissue of a thickness that prevents detection of the fluorescence light by means of the fluorescence detection apparatus.

In a preferred embodiment, the system is further adapted for correcting or improving an optical or medical image taken during surgery based on information derived from said 3D patient data set. For example, the information derived from said 3D patient data set may include one or both of absorption properties or information regarding a tissue composition, and this information may be used for accounting for absorption of fluorescence light and/or improving multispectral optical or photoacoustic images.

While in the embodiments described above, the aim was to introduce information from the 3D patient data set in order to augment the view on or an image of the patient's body, or to improve an optical a medical image taken during surgery, the system may also operate the other way around, thereby allowing to introduce information gathered from the surgery to the 3D patient data set. For example, when certain clinical observations are made during surgery, an important question will be whether this clinical observation could already have been derived from the pre-operative 3D patient data set. Based on knowledge gathered during surgery, the 3D patient data set can be enriched and be used for future scientific or educational purposes. Accordingly, in one embodiment, the system is further configured to identify certain locations viewed in the actual patient by means of the visualization tool and to associate these locations with the corresponding location in the 3D patient data set. Simply speaking, this provides technical means allowing for matching what is "seen" at a certain location during surgery with the corresponding location in the 3D patient data set. For this purpose, the system may comprise a user interface allowing a user to input information associated with said corresponding locations in said 3D patient data set. This information could be images taken during surgery, but could also include e.g. the results of a biopsy, for example revealing whether a certain suspicious tissue was cancerous or not.

### SHORT DESCRIPTION OF THE FIGURES

- Fig. 1: is a schematic view illustrating administering of a fluorescence marker to a patient and determining the markers' positions in a medical image.
- Fig. 2: is a schematic in view of a marker comprising a radioactive tracer, a fluorescent dye and a binding motive for binding with an enzyme of a prostate cell.
- Fig. 3: schematically illustrates the generation of a fluorescence marker comprising nano colloids, a radioactive tracer and a fluorescent dye.
- Fig. 4: is a schematic representation of a workflow in which fluorescence markers are attached in a patient's body using an adhesive and the location is determined using medical imaging.
- Fig. 5: is a schematic representation of a workflow in which the locations of fluorescence markers in a patient's body and in a medical image are manually chosen.
- Fig. 6: shows a laparoscope and an organ under surgery, as well as a visual and a fluorescence image recorded therewith.
- Fig. 7: shows a fluorescence camera and an RGB camera employed under surgery, as well as a visual and a fluorescence image recorded therewith.
- Fig. 8: shows a top view and a front view of glasses equipped with a fluorescence camera.
- Fig. 9: is a set of illustrative drawings explaining the derivation of a spatial relationship between a visualization tool and a patient's body.
- Fig. 10: illustrates the spatial relationship between a virtual laparoscope and a medical image, and further shows an image taken with the laparoscope that is augmented with information from the medical image.
- Fig. 11: schematically shows a workflow in which absorption properties of tissue derived from a medical image are used to correct a fluorescence image.
- Fig. 12: is a schematic diagram illustrating how information gained under surgery can be used to enrich a pre-operative medical image.

### DESCRIPTION OF THE PREFERRED EMBODIMENTS

For the purposes of promoting an understanding of the principles of the invention, reference will now be made to preferred embodiments illustrated in the drawings, and specific language will be used to describe the same. It will nevertheless be understood that no limitation of the scope of the invention is thereby intended, such alterations and further modifications in the illustrated system and such further applications of the principles of the invention as illustrated therein being contemplated as would normally occur now or in the future to one skilled in the art to which the invention relates.

### 1.) Administering fluorescence markers and determining their position in a 3D patient data set

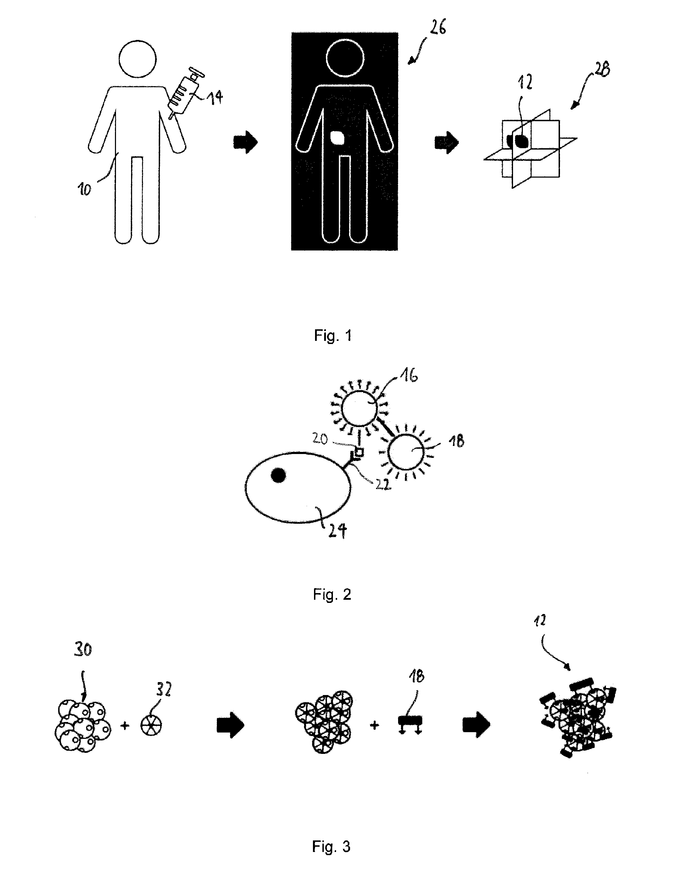

With reference to Figures 1 to 5, it will first be described how fluorescence markers may be administered to a patient, and how the spatial position of the fluorescence markers within the body can be included in or associated with a 3D patient data set.

Figure 1 is a schematic illustration of a corresponding procedure. Figure 1 shows a patient 10 to which a fluorescence marker 12 is administered by injection using a syringe 14. An example of a marker 12 that can be used for this purpose is shown in Figure 2. The marker 12 comprises a radioactive tracer and a fluorescent dye 18, for example ICG. The radioactive tracer comprises chelator complex 16 with a radiometal such as ⁶⁸Ga, which is connected to the fluorescent dye via a linker. Both are connected to a binding motive 20 which binds with a PSMA enzyme 22 of a prostate cell 24. Accordingly, when the marker 12 is injected to the patient 10 as shown in Figure 1, the marker 12 will bind with the PSMA enzyme 22.

With reference again to Figure 1, after the marker 12 has been injected and has accumulated close to prostate cells 24, a medical image is taken, as indicated by reference sign 26. The medical image can for example be a combined PET-CT image which allows for obtaining a 3D image of the body using a CT scan, and in addition to detect the localization of the marker 12 by means of the radioactive tracer using the PET modality. As a result of this, a medical image 28 of the patient is obtained, which includes, in addition to the medical image proper, information regarding the spatial position of the fluorescence markers 12 within the body of the patient 10. This medical image 28 is an example of the "3D patient data set" referred to in the summary of the invention above. In the Figures, the medical image 28 is symbolically resembled by a corresponding coordinate system. Also included in the representation of the medical image is information about the spatial location of the markers 12, which for simplicity is designated by the same reference sign 12, although the skilled person will appreciate that this is not the actual, physical marker, but the representation of the marker in the medical image 28.

Figure 3 schematically shows the construction of an alternative fluorescence marker 12 in a way similar to that described in van den Berg et al. "Fluorescence Guidarrce in urologic surgery ", Current opinion in urology 22, 2; p. 109-120 (2012*).* The fluorescence marker 12 of Figure 3 consists of nano colloids 30 which are combined with a radio tracer 32, which in the example shown is formed by ⁹⁹Tc. Alternatively, depending on the imaging modality to be used, a CT or MR imaging (MRI) contrast agents could be used instead. This combined nano colloid and radioactive tracer 30, 32 is then combined with a fluorescent dye 18, in the present example indocyanine green ICG, such as to form a nano particle complex of radio tracer and fluorescent dye. When a medical image 28 is taken, the location of the fluorescence marker 12 within the image can be discerned due to the radioactive tracer 30. Unlike the example of Figure 2, upon injection, the marker 12 will not accumulate at a specific location, but a certain spatial distribution of fluorescence markers 12 within the body will nevertheless be obtained, and the information regarding the spatial position of the fluorescence markers 12 within the body can be obtained and included in or associated with the medical image 28.

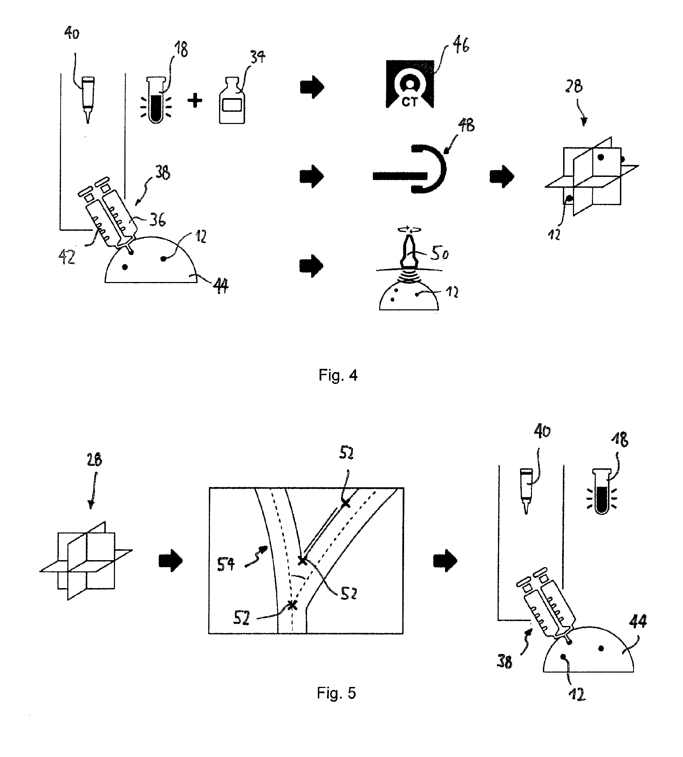

Figure 4 symbolically represents a further scenario for administering fluorescence markers 12 and determining the position in the medical image 28. In the scenario of Figure 4, a mixture of a fluorescent dye 18 and a contrast agent 34 is given into a first barrel 36 of a double barrel syringe 38, and an adhesive 40 is given into a second barrel 40 of said double barrel syringe 38. With the double barrel syringe 38, a mixture of the fluorescent dye 18, contrast agent 34 and adhesive can be applied directly to a body part, such as an organ 44, as illustrated in the left part of Figure 4. Localized spots of the mixture of fluorescent dye 18, contrast agent 34 and adhesive 40 can be regarded as "fluorescence markers" 12 in the sense of the present invention. In the example shown, the adhesive 40 is a biodegradable adhesive, such as a fibrin adhesive or cyanoacrylate.

Next, a medical image 28 of the body or body part including said fluorescence markers 12 is taken. As is indicated schematically in Figure 4, this medical image can be taken using a conventional CT apparatus 46, or a so-called "Dyna CT" apparatus 48 comprising a C-shaped gantry. The "Dyna CT" apparatus 48 allows to take the medical image 28 not only pre-operatively, but also during the surgery. Note that if the fluorescent dye 18 is ICG, some adhesives 40 such as cyanoacrylate tend to suppress the quenching effect thereof, which leads to a longer-lasting fluorescence. This means that the fluorescence markers 12 can be applied preoperatively and still lead to sufficient fluorescence during surgery. Moreover, the fluorescence markers consisting of ICG and cyanoacrylate show a stronger fluorescence signal as compared to the fluorescent dye in solution.

In some embodiments, the medical image 28 can also be obtained by 3D ultrasound imaging or photoacoustic tomography (PAT) imaging. This is indicated in Figure 4, where an ultrasound probe head 50 is schematically shown. When using ultrasound imaging, it may be difficult to discern the fluorescence markers 12 in the image. In this case, it would be possible to identify the locations of the markers 12 by touching their locations with the tip of a needle which can be seen in the ultrasound image, and this spatial information can then be transferred to the ultrasound image. In case of photoacoustic tomography, when choosing the photo acoustic wavelength properly, the fluorescence marker 12 can be directly detected or "seen" in the image.

In alternative embodiments, it is also possible to "manually" select both, the fluorescence marker locations in the medical image 28 and in the actual patient's body 10. This variant is illustrated with reference to Figure 5. In Figure 5, the starting point is a 3D medical image 28, which however does not yet include any information about spatial positions of fluorescence markers. This patient information is added to the medical image "manually", i.e. the surgeon may mark certain characteristic locations 52 in the medical image, as shown in the middle of Figure 5. The "characteristic locations" are locations which the surgeon will also reliably find in the patient's actual body for manually applying markers 12 thereto, for example by again using an adhesive 40 and a fluorescent agent 18, but no contrast agent (see right part of Figure 5). In the example shown in Figure 5, the characteristic locations 52 could for example correspond to a branching point of a blood vessel 54 and a further position located a predetermined distance away from the branching point on a predetermined one of the branches of the blood vessel 54. Generally, there are no limitations about the pattern of the distribution of the fluorescence markers 12, as long as the surgeon is able to determine precisely where these locations are both in the 3D medical image 28 and in the actual patient's body 10. This variant has the advantage that after placing the markers 12, no additional medical imaging step is necessary, does however require a certain amount of skill and experience of the surgeon.

### 2.) Detecting fluorescence

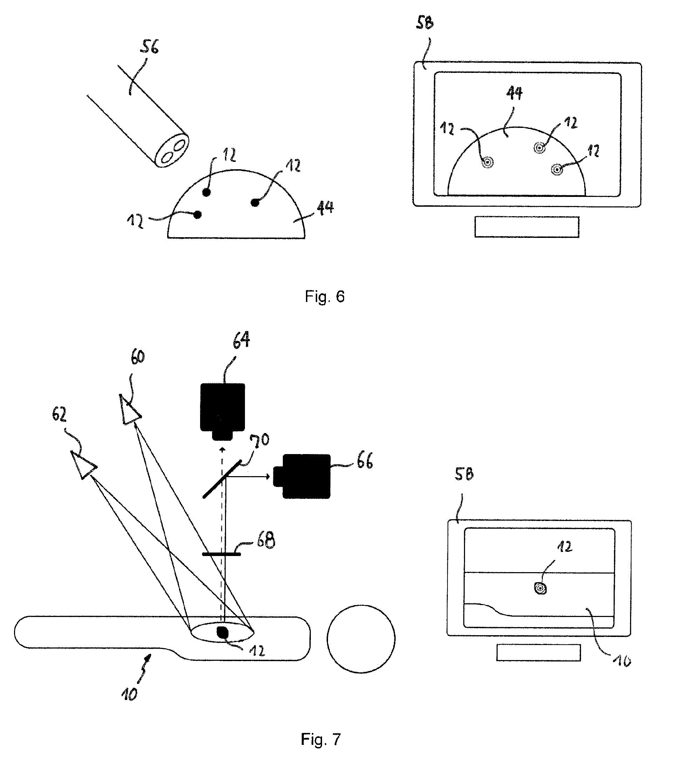

As explained in the summary of the invention, the system of the invention comprises an apparatus for detecting fluorescence from the fluorescence markers 12 provided in the patient's body 10. Examples of such apparatuses and methods for using the same will be illustrated with reference to Figures 6, 7 and 8.

Figure 6 shows a laparoscope 56 that can be used intraoperatively, as well as an organ 44 to which fluorescence markers 12 are attached. The laparoscope 56 has both, a visual image modality, as well as a fluorescence image modality. In the example shown, the laparoscope 56 has a light source (not shown) for illuminating the surgical site, such as the organ 44 with visual light, a light source (not shown) for emitting excitation light for exciting the fluorescent dye included in the fluorescence markers 12, as well as corresponding image sensors for recording video images of the visible light and the fluorescence light. For this purpose, the laparoscope 56 could have two different image sensors and a wavelength selective splitter, or could have a single image sensor and a switchable wavelength selective filter.

Both, the visual image and the fluorescence image can then be displayed in an overlayed fashion on a display shown under the reference sign 58 in Figure 6. On the display 58, the image of the organ 44 and the fluorescence markers 12 are designated with the same reference signs as the corresponding physical objects. Note that typically, the fluorescent dye included in the fluorescence marker 12 will emit in the NIR spectral range, which is almost not or not seen with an ordinary camera nor with the naked eye, but the fluorescence markers 12 can of course be visualized on the display 58 such as to be seen by the surgeon. Note that the image sensor (not shown) for recording video images of the visible light, together with the display 58, forms an example of the "visualization tool" referred to in the summary of the invention. Moreover, the image sensor (not shown) for recording fluorescence images forms an example of the "apparatus for detecting fluorescence" referred to in the summary of the invention. Note that in some embodiments, the fluorescence markers are not displayed on the display 58, but only used for registration purposes.

In Figure 7, and alternative apparatus for detecting fluorescence from the fluorescence markers 12 is shown. Figure 7 shows again a patient's body 10 including a fluorescence marker 12. While reference is made to "the fluorescence marker 12" for simplicity, in actual examples there could be a fluorescent region in which functionalized markers such as the markers shown in Fig. 2 have accumulated in a target tissue. Further shown in Figure 7 are a light source 60 for emitting excitation light for exciting fluorescence of a fluorescent dye included in the fluorescence marker 12, and a light source 62 for visual light that illuminates the region of interest. This light source 62 could simply be the ordinary lighting system of the operating room. Further shown in Figure 7 is a fluorescence camera 64 for taking fluorescence images, typically IR images, and an ordinary RGB camera 66 for taking images in the visual spectrum.

In Figure 7, the fluorescence light is symbolized by a dashed ray, while the visible light is illustrated by a solid ray. In the light path of both, the visible and the fluorescence light, a lens 68 is provided. A wavelength selective splitter 70 is provided for reflecting the visible light to the RGB camera 66 and for transmitting the IR light to the fluorescence camera 64. Instead of a wavelength selective splitter 70, a wavelength unselective half mirror could be used, and the fluorescence camera 64 and the RGB camera 66 could be provided with suitable filters transmitting only the desired component of the available light. Again, a display 58 is shown on which the RGB image of the body and the fluorescence image of the fluorescence marker 12 (or tissue in which fluorescence markers 12 are accumulated) are superimposed. In the embodiment shown in Figure 7, the RGB camera 66, together with the display 58, forms an example of the "visualization tool" referred to in the summary of the invention, while the fluorescence camera 64 resembles an example of an "apparatus for detecting fluorescence".

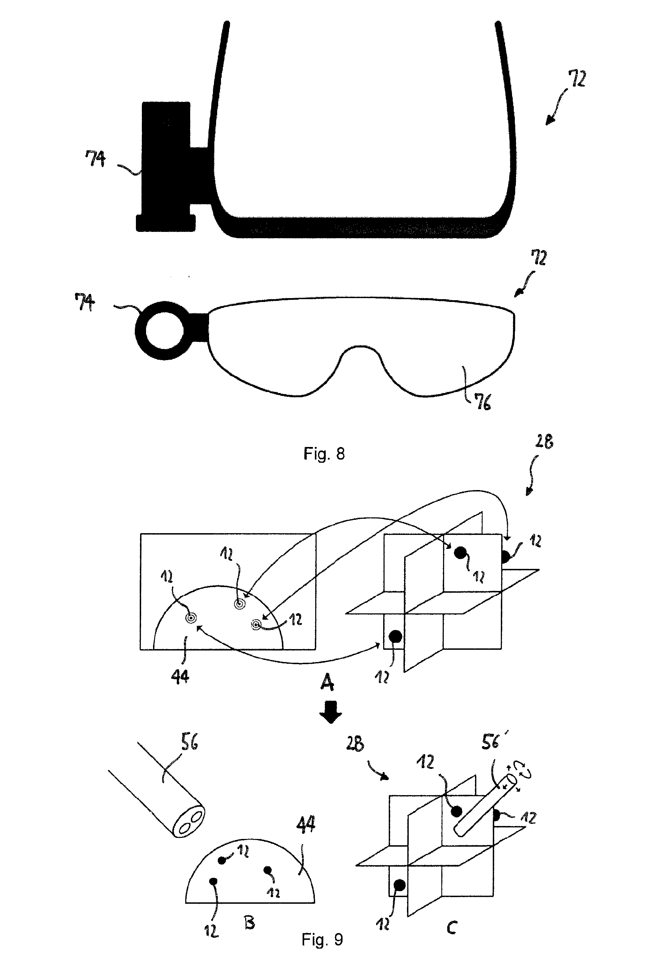

Figure 8 shows a yet further embodiment, where the apparatus for detecting fluorescence is formed by glasses 72 shown in a top and a front view, respectively. On the glasses 72, a fluorescence camera 74 is provided which allows for taking fluorescence images of the fluorescence markers 12 distributed inside the patient's body 10 under surgery. Through a lens 76 of the glasses, the user can get a direct view on the surgical site. Moreover, additional graphical information can be projected into the field of view of the user. This additional graphical information could represent augmented reality to be described below, but also the fluorescence image detected by the fluorescence camera 74. Namely, since there is a fixed spatial relationship between the lens 76 of the glasses and the fluorescence camera 74 attached to the glasses 72, the fluorescence image taken by the fluorescence camera 74 can be correlated with the field of view seen by the user wearing the glasses 72. The glasses 72 form a further example of a "visualization tool" as referred to in the summary of the invention, while the fluorescence camera 74 attached thereto forms a yet further example of the "apparatus for detecting fluorescence". Instead of projecting the additional graphical information into the glasses, such graphical information could alternatively be projected onto the patient's body.

### 3.) Deriving a spatial relationship between visualization tool and patient's body

In order to augment a view or image of said patient's body 10 with information derived from the 3D patient data set, i.e. the medical image 28, it is necessary to derive a spatial relationship between the visualization tool and the patient's body 10. This derivation is based on spatial information of the fluorescence markers 12 which is included in the medical image 28 and which can be obtained using the apparatus for detecting fluorescence under surgery. This is further illustrated with reference to Figure 9. Note that while in the described embodiment, the fluorescence markers are essentially point-like objects, in further embodiments the fluorescence markers can be used to mark surfaces, or surface parts, that can be surface registered. In addition, the registration need not exclusively rely on the fluorescence markers. For example, the markers can be combined with surface registration, where a surface of the patient's body part is matched with a surface within the 3D data set. In this case biomedical modelling algorithms can be employed for deformable registration, in which the point or surface correspondences serve as boundary conditions.

Figure 9B shows a situation under surgery, where again an organ 44 provided with fluorescence markers 12 and a laparoscope 56 are shown. Using the fluorescence camera (not shown) of the laparoscope 56, a fluorescence image can be recorded which includes the fluorescence image of the fluorescence markers 12, as shown in the left half of Figure 9A. This view of the fluorescence markers 12 can be correlated with the spatial distribution of fluorescence markers 12 in the medical image 28, schematically shown in the right half of Figure 9A. This correlation is symbolically represented by the double arrows associating the fluorescence image of the fluorescence markers 12 and the fluorescence marker 12 as included in the medical image 28. Using per se known tracking algorithms, it is then possible to determine the position of the fluorescence camera of the laparoscope 56 with regard to the patient coordinate system, as is shown in Figure 9C. Further, since there is a known spatial relationship between the fluorescence camera and the visualization tool, i.e. the RGB camera likewise included in the laparoscope 56, also the position of the visualization tool with respect patient coordinate system, or in other words, a spatial relationship between the visualization tool and the patient's body 10, can be derived.

The way this is done practically is that a virtual laparoscope 56' is moved around in the coordinate system of the medical image 28 until the fluorescence image currently recorded by the fluorescence camera of the laparoscope 56 is in agreement with a calculated fluorescence image of the virtual laparoscope 56' within the medical image 28. For this purpose, as mentioned in the summary of the invention, an inside-out-tracking based on it 2D to 3D registration can be employed, in which the 2D fluorescence image is matched with the known 3D positions of the fluorescence markers 12 in the three-dimensional medical image 28.

Alternatively, if the laparoscope 56 has the capability of obtaining 3D information of the location of the fluorescence markers 12, for example by employing two cameras placed at a distance for obtaining stereo data, then this 3D information can be matched with the known 3D positions of the fluorescence markers 12 within the 3D medical image 28. In this case, the 3D medical image can be mapped into a coordinate system of the laparoscope 56, possibly accounting for deformation of non-rigid body parts, e.g. using a biomechanical model.

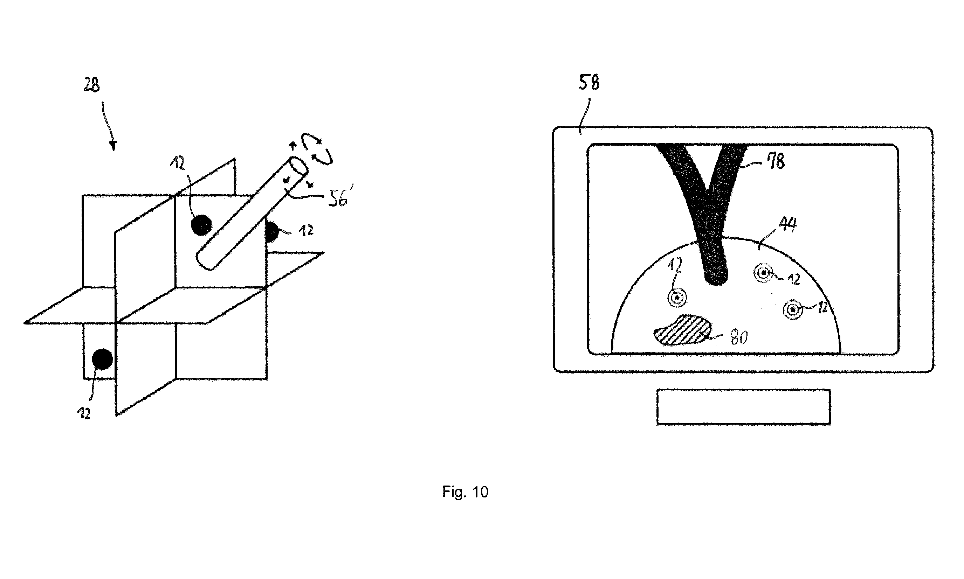

Figure 10 shows in the left half the situation, where the position of the laparoscope 56 with regard to the patient body 12, or, in other words, the position of the virtual laparoscope 56' with regard to the medical image 28 has been derived. In this case, it becomes possible to augment the RGB image of the laparoscope 56, as shown in the right hand of Figure 6, with information derived from the medical image 28. In the example shown in Figure 10, this augmentation amounts to displaying a blood vessel 78, which in the actual RGB image (see right half of Figure 6) cannot be seen, for example because it is covered by other tissue. By projecting the blood vessel 78 into the image, the surgeon can be prevented from inadvertently injuring the same. Note that the information that the blood vessel 78 is there is derived from the medical image 28 which includes the blood vessel 78. A further example of this type of "augmented reality" is a tumor 80, or what is believed to be at tumor based on the medical image 28. In a preferred embodiment, the surgeon can select features in the medical image 28 prior to or during the surgery that are to be projected into the image shown on display 58.

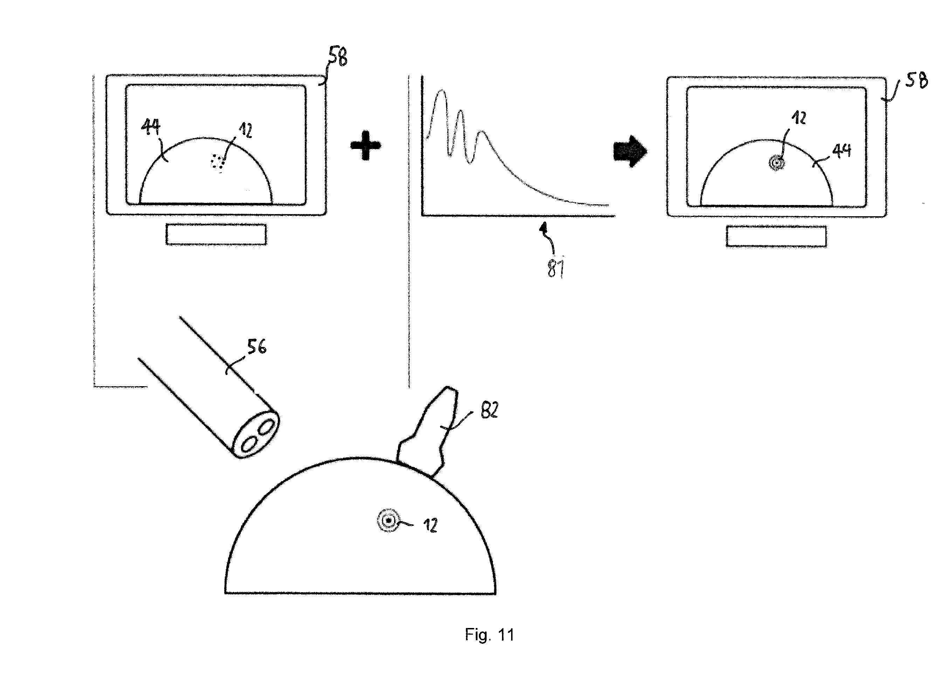

The information derived from the medical image 28 can however not only be used to augment the view or image of the patient's body 10, but also to correct or improve an optical or medical image taking during surgery. An example for this is discussed with reference to Figure 11. As is seen on the left of Figure 11, and RGB image and a fluorescence image are taken using a laparoscope 56, and the combined image is displayed on the display 58. As is schematically indicated in Figure 11, the fluorescence image may appear somewhat feeble, for example due to the fact that the corresponding fluorescence marker 12 is covered by tissue, such that a substantial part of the fluorescent light is absorbed and scattered thereby. Accordingly, there is some uncertainty whether actually a fluorescence marker 12 is detected, or whether there is an artifact.

Based on the 3D medical image 28, the thickness of the tissue covering the fluorescence marker 12 can be determined, and from this the expected absorption of fluorescence light can be estimated. This estimation can be made more precise if the absorbance of the tissue is accounted for, which can in many cases likewise be derived from the medical image 28. In the example shown in Figure 11, the medical image is, at least in part, obtained by photoacoustic tomography, which allows for deriving a characteristic absorption curve as shown at reference sign 81 in Figure 11. A photoacoustic tomography probe head 82 is shown in the lower part of Figure 11. Based on the knowledge about the thickness of the tissue covering the fluorescence marker 12 and its characteristic absorption, the loss of intensity of the fluorescence light detected can be corrected, as is symbolically shown on the right of Figure 11, where the brightness of the fluorescence image is correspondingly increased and a fluorescence marker can be identified indeed.

While in the example shown in Figure 11, and absorption curve is obtained using photoacoustic tomography, similar or related information can also be derived if other medical imaging modalities are used. For example, if the medical image 28 is based on MRI imaging, the tissue composition can likewise be characterized, and the characteristic absorption can be estimated therefrom. The absorption properties are not only important for evaluating the fluorescence light from the fluorescence markers 12, but can also be used when multispectral or photoacoustic images are taken for diagnostic purposes.

In addition, by assessing tissue properties from a pre-operatively taken 3D medical image, the quality of a photoacoustic tomography image taken during surgery can be improved.

### 4.) Further functionalities

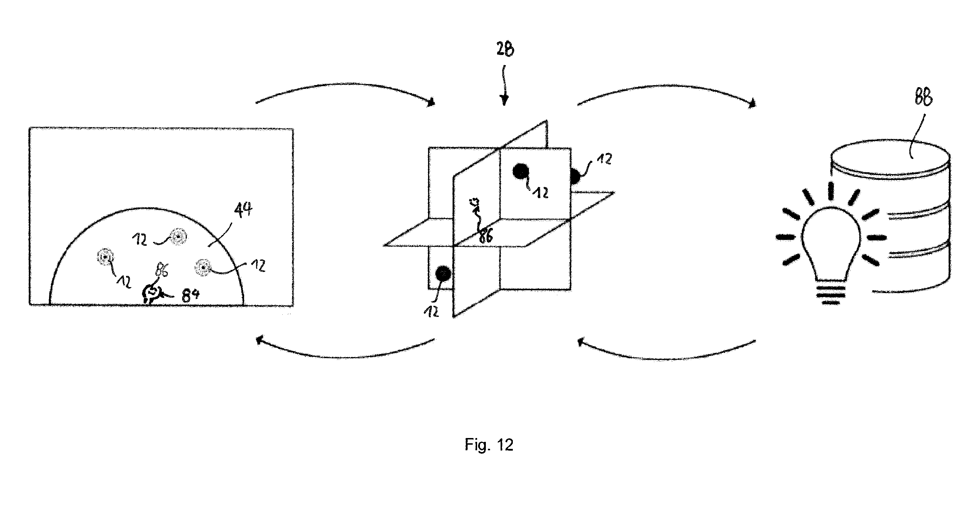

While in the embodiments described above, the main focus was on adding information from the medical image 28 to the image or view of the patient 10 as provided by the visualization tool, the system of the invention may also operate the other way around in the sense that information gathered during surgery is associated with the three-dimensional medical image 28. Based on knowledge gathered during surgery, the medical image 28, or more generally, the 3D patient data set, can be enriched and be used for future scientific or educational purposes. This is schematically illustrated in Figure 12 which on the left shows an image taken during surgery, showing again the organ 44 and the fluorescence markers 12, and in the middle symbolically represents the medical image 28. Consider that the surgeon takes several tissue samples during the intervention. Each time a sample is taken, the surgeon inputs a command that indicates to the system that the location 86 of a biopsy tool 84 used for this purpose should be recorded, and this location 86 may be labeled. Each of these labeled locations 86 can then be associated with the medical image 28, as is indicated in the middle of Figure 12.

Then, if the biopsy has been made the subject of a histological analysis, the result thereof can likewise be associated with the labeled location 86 in the medical image 28. The medical image 28 enriched by this additional histologic information can then be stored in a database shown in the right of Figure 12 under reference sign 88. Then, anyone searching in the database 88 for certain histological findings can find corresponding medical images 28, and can thereby learn how this histology was manifested in the medical image. This way, doctors and algorithms can better learn how to interpret medical images 28.

Clearly, this is just one example how the medical image 28 can be enriched, and in general, all types of information obtained during surgery can this way be associated with corresponding locations in the medical image 28, including pictures or video clips taken during surgery. According to preferred embodiments of the invention, the system provides an interface allowing a user to input information associated with corresponding locations in the medical image 28.

Although a preferred exemplary embodiment is shown and specified in detail in the drawings and the preceding specification, these should be viewed as purely exemplary and not as limiting the invention. It is noted in this regard that only the preferred exemplary embodiment is shown and specified, and all variations and modifications should be protected that presently or in the future lie within the scope of protection of the invention as defined in the claims.

### List of References

- 10: patient
- 12: fluorescent marker
- 14: syringe
- 16: chelator complex
- 18: fluorescent dye
- 20: binding motive
- 22: PSMA enzyme
- 24: prostate cell
- 26: medical imaging step
- 28: medical image
- 30: nano colloide
- 32: radioactive tracer
- 34: contrast agent
- 36: first barrel of double syringe 38
- 38: double barrel syringe
- 40: adhesive
- 42: second barrel of double barrel syringe 38
- 44: organ
- 46: CT apparatus
- 48: Dyna CT apparatus
- 50: ultrasound probe head
- 52: characteristic locations
- 54: blood vessel
- 56: laparoscope
- 56': virtual laparoscope
- 58: display
- 60: excitation light source
- 62: visual light source
- 64: fluorescence camera
- 66: RGB camera
- 68: lens
- 70: wavelength selective splitter
- 72: glasses
- 74: fluorescence camera
- 76: lens
- 78: blood vessel
- 80: tumor
- 81: absorption curve
- 82: photo acoustic tomography probe head
- 84: biopsy tool
- 86: location
- 88: database

## Claims

1. A system for fluorescence aided surgery, comprising:
- a storage medium or a data connection with a storage medium storing a 3D data set (28), in particular a medical image, of a patient (10), wherein information regarding a spatial position of one or more fluorescence markers (12) within the body of said patient (10) is included in or associated with said 3D patient data set,
- a visualization tool (66, 58; 72) allowing for augmenting a view or an image of said patient's body (10) with information derived from said 3D patient data set (28),
- an apparatus (64, 74) for detecting fluorescence from said one or more fluorescence markers (12) provided in said patient's body (10), and
- a computation device adapted to derive a spatial relationship between said visualization tool (66, 72) and the patient's body (10), based at least in part on
• said detected fluorescence of said one or more markers (12),
• said information regarding said spatial position of said one or more fluorescence markers (12) within the body (10) of said patient included in or associated with said 3D patient data set (28), and
• a known spatial relationship between the apparatus (64, 74) for detecting fluorescence and said visualization tool (66, 72).

2. The system of claim 1, wherein the fluorescence marker (12) emits NIR light, and/or wherein the fluorescence marker (12) is biodegradable.

3. The system of one of the preceding claims, wherein said visualization tool comprises a camera (66) and a display (58) for displaying images taken with said camera, glasses (72), or a projector for projecting images or visual information onto the patient's body.

4. The system of one of the preceding claims, further comprising at least one fluorescence marker (12) for placing in the body of the patient (10), wherein said fluorescence marker preferably further includes a contrast agent or radioactive tracer (32) for medical imaging, in particular for CT, PET, SPECT, MR, ultrasound or photoacoustic imaging.

5. The system of claim 4, wherein said fluorescence marker (12) further comprises a ligand or binding motive for selectively binding to target cells, wherein said ligand or binding motive is preferably selected from the group consisting of peptide, protein, biopolymer, synthetic polymer, anti-gene, anti-body, microorganism, virus, receptor, enzyme, hormone, chemical compound, toxin, surface modifier and combinations thereof.

6. The system of one of claims 4 or 5, wherein said fluorescence marker (12) is included in a liquid for injection to the patient's body (10), or in a pill for oral administering.

7. The system of one of claims 4 or 5, wherein said fluorescence marker (12) is provided with an adhesive (40) agent or binder, in particular a biodegradable adhesive agent or binder, and preferably a fibrin adhesive agent or cyanoacrylate, and/or wherein said fluorescence marker (12) comprises nanoparticles comprising a compound of said medical imaging contrast agent or radioactive tracer (32) and a fluorescent dye (18), wherein said system preferably further comprises a double barrel syringe (38), one barrel (36) including a fluorescence marker and one barrel (42) including said adhesive.

8. The system of one of the preceding claims, wherein said apparatus for detecting fluorescence from said one or more fluorescence markers comprises one or more of a laparoscopic fluorescence imaging device (56), a fluorescence imaging camera (64) or fluorescence imaging glasses (72).

9. The system of one of the preceding claims, wherein said computation device is adapted to derive said spatial relationship between said visualization device (56, 66, 72) and the patient's body (10), based on one or more of the following:
- an 2D/3D registration algorithm, in which a 2-D image of one or more fluorescence markers (12) is correlated with known 3D positions of said fluorescence markers (12) in said 3D patient data set (28),
- a 3D/3D registration algorithm, in which 3D information of the location of said fluorescence markers (12) is obtained using said apparatus (64, 74) for detecting fluorescence, and said 3D information is matched with known 3D positions of said fluorescence markers (12) in said 3D patient data set (28), and/or wherein said apparatus (64, 74) for detecting fluorescence comprises two cameras placed at a distance for obtaining stereo data, and/or wherein said apparatus for detecting fluorescence (64, 74) is adapted to analyze shadows and/or motions and to derive 3D positions of the fluorescence markers (12) therefrom, or to measure 3D positions of the fluorescence markers (12) based on time of flight measurements.

10. The system of one of the preceding claims, wherein said system is further adapted for augmenting the view or image of said patient's body with one or more of
- target structures, such as tumors (80), which have been previously marked in the 3D patient data set,
- structures at risk, which are to be saved from injuries, in particular blood vessels (78) or nerves,
- landmarks assisting the surgeon in orientation, in particular organs or bones,
- structures which are covered by tissue,
- fluorescence markers (12), in particular markers with fluorescence outside the visible part of the spectrum and/or markers that are covered with tissue of a thickness that prevents detection of the fluorescence light by means of the fluorescence detection apparatus (64, 74).

11. The system of one of the preceding claims, wherein said system is further adapted for correcting or improving an optical or medical image taken during surgery based on information derived from said 3D patient data set (28), wherein said information derived from said 3D patient data set preferably includes one or both of absorption properties or tissue composition information, and wherein this information is used for accounting for absorption of fluorescence light and/or improving multispectral images.

12. The system of one of the preceding claims, further configured to identify certain locations (86) viewed in the actual patient (10) by means of the visualization tool (66, 72) and to associate these locations with the corresponding location in the 3D patient data set (28), whereinthe system preferably further comprises a user interface allowing a user to input information associated with said corresponding locations (86) into said 3D patient data set.

13. A method of augmenting a view or an image of a patient's body (10), comprising the steps of:
- providing a 3D data set (28), in particular a medical image, of the patient (10), wherein information regarding a spatial position of one or more fluorescence markers (12) within the body (10) of the patient is included in or associated with said 3D patient data set (28),
- detecting fluorescence from said one or more fluorescence markers (12) provided in the patient's body (10),
- computing a spatial relationship between a visualization tool (66, 72) and the patient's body (10) at least in part based on
• said detected fluorescence of said one or more markers (12),
• said information regarding said spatial position of said one or more fluorescence markers (12) within the body (10) of said patient included in or associated with said 3D patient data set (28), and
• a known spatial relationship between the apparatus (64, 74) for detecting fluorescence and said visualization tool (66, 72), and
- augmenting the view or the image of the patient's body (10) with information derived from said 3D patient data set (28) using said visualization tool (66, 72).

14. The method of claim 13, wherein said step of augmenting the view or the image of the patient's body (10) with information derived from said 3D patient data set (28) comprises one or more of
- displaying said information derived from said 3D patient data set (28) together with a camera image of the patient's body (10) on a display (58),
- projecting said information derived from said 3D patient data set (28) onto glasses (72) worn by the surgeon, or
- projecting said information derived from said 3D patient data set (28) onto the patient's body (10), and/or wherein said step of computing said spatial relationship between said visualization device (56, 66, 72) and the patient's body (10), involves one or more of the following:
- a 2D/3D registration algorithm, in which a 2D image of one or more fluorescence markers (12) is correlated with known 3D positions of said fluorescence markers (12) in said 3D patient data set (28),
- a 3D/3D registration algorithm, in which 3D information of the location of said fluorescence markers (12) is obtained using said apparatus (64, 74) for detecting fluorescence, and said 3D information is matched with known 3D positions of said fluorescence markers (12) in said 3D patient data set (28).

15. The method of claim one of claims 13 or 14, wherein said step of augmenting the view or image of said patient's body (10) comprises augmenting the same with one or more of
- target structures, such as tumors (80), which have been previously marked in the 3D patient data set,
- structures at risk, which are to be saved from injuries, in particular blood vessels (78) or nerves,
- landmarks assisting the surgeon in orientation, in particular organs or bones,
- structures which are covered by tissue,
- fluorescence markers (12), in particular markers with fluorescence outside the visible part of the spectrum and/or markers that are covered with tissue of a thickness that prevents detection of the fluorescence light by means of the fluorescence detection apparatus (64, 74), and/or in which a system according to one of claims 1 to 12 is used.
